Europäisches Patentamt

European Patent Office

Office européen des brevets

(19)

(11) Veröffentlichungsnummer: **0 031 299**
**B1**

(12) **EUROPÄISCHE PATENTSCHRIFT**

(45) Veröffentlichungstag der Patentschrift:
13.02.85

(21) Anmeldenummer: **80810388.1**

(22) Anmeldetag: **15.12.80**

(51) Int. Cl.⁴: **C 07 C 143/60**

(54) Verfahren zur Herstellung von 2-Amino-1-naphthalinsulfonsäure.

(30) Priorität: **20.12.79 CH 11315/79**

(43) Veröffentlichungstag der Anmeldung:
**01.07.81 Patentblatt 81/26**

(45) Bekanntmachung des Hinweises auf die Patenterteilung:
**13.02.85 Patentblatt 85/7**

(84) Benannte Vertragsstaaten:
**CH DE FR GB IT LI**

(56) Entgegenhaltungen:
**DE - A - 2 831 956**
**DE - A - 2 831 965**
**DE - A - 2 831 966**
**DE - A - 2 831 992**
**DE - A - 2 831 993**
**DE - A - 2 831 994**
**DE - A - 2 831 995**
**GB - A - 436 464**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Plattner, Eric, Prof. Dr., Jurastrasse 18, CH-4411 Seltisberg (CH)**
Erfinder: **Stäubli, Sebastian, Lanzenbergstrasse 17, CH-4465 Magden (CH)**
Erfinder: **von Kaenel, Fred, Jurastrasse 19, CH-4411 Seltisberg (CH)**

## Beschreibung

Gegenstand der Erfindung ist ein verbessertes Verfahren zur Herstellung von 2-Amino-1-naphthalinsulfonsäure (Tobias-Säure), im weiteren (TS) genannt. Das verbesserte Verfahren zur Herstellung von TS erlaubt es eine TS herzustellen, die über einen nur sehr geringen Gehalt an 2-Aminonaphthalin verfügt und ist durch hohe Raum-Zeit-Ausbeuten ausgezeichnet.

Das vorliegende Verfahren zur Herstellung von TS geht von 2-Hydroxynaphthalin aus, im weiteren (HN) genannt und durchläuft folgende Hauptreaktionsstufen:

a) die Sulfonierung zu 2-Hydroxynaphthalin-1-sulfonsäure im folgenden als Oxy-Tobiassäure (OTS) bezeichnet, mittels Chlorsulfonsäure in einem reaktionsinerten organischen Lösungsmittel;

b) der Neutralisation der freigesetzten und noch im Reaktionsmedium vorhandenen Salzsäure bzw. überschüssigen Chlorsulfonsäure und Umwandlung der entstandenen 2-Hydroxynaphthalin-1-sulfonsäure in das entsprechende Ammoniumsalz mittels Ammoniak;

c) der Bucherer-Reaktion zur Umwandlung der 2-Hydroxy- in die 2-Amino-Gruppe mittels Ammoniak und

d) die anschließende Ausfällung der entstandenen TS mittels verdünnter Schwefelsäure.

Die Bucherer-Reaktion wird üblicherweise im Rahmen der TS-Herstellung durch Aminierung von Alkalimetallsalzen der 2-Hydroxy-1-naphthalinsulfonsäure auch im industriellen Rahmen angewandt.

Bei dieser Reaktion kommt es zur Bildung geringer Mengen an Nebenprodukten. Eines dieser Nebenprodukte ist 2-Aminonaphthalin im weiteren (AN) genannt, das eine carzinogene Verbindung darstellt.

Die Occupational Safety and Health Administration (OSHA) hat Verordnungen erlassen, die eine Handhabung von AN enthaltenden Materialien nur dann erlauben, wenn der AN-Gehalt 0,1 Gewichtsprozent oder weniger ausmacht [siehe: The Federal Register 39, No. 20, Teil III, Seiten 3756—3797 (1974), und »The Control of Industrial Bladder Tumours«, Scott and Williams, Brit. J. Industrial Medicin 14, Seiten 150—163 (1957)]. Die großtechnisch hergestellte TS mußte bisher aufwendigen Nachbehandlungen unterzogen werden, damit sie obigem Erfordernis entspricht, oder aber die Produktion hätte aufgegeben werden müssen. Es wurde nun gefunden, daß wenn man die Gesamtreaktion anstatt auf die Bildung von Alkalimetallsalzen in den Reaktionszwischenstufen, auf die besser löslichen Ammoniumsalze ausrichtet, überraschenderweise im Endprodukt, der TS, nur sehr geringe Mengen 2-Naphthylamin erhalten werden, die weit unter den geforderten Grenzen liegen.

Die bessere Löslichkeit des Ammoniumsalzes der OTS gestattet es, die Reaktionsansätze in höheren Konzentrationen als mit Alkalisalzen zu führen, wodurch wie bereits genannt, bessere Raum-Zeit-Ausbeuten erzielt werden und im Zusammenhang damit bessere Investitions-, Energie-, Ökologienutzung, um nur einige vorteilhafte Aspekte zu nennen.

Die Herstellung von Alkalimetallsalzen von OTS durch Sulfonierung von HN mit Chlorsulfonsäure ist bekannt. Im einzelnen wird hierzu auf US-PS 1 716 082 und Fierz-David and Blangey, »Fundamental Processes of Dye Chemistry« (Interscience Publishers, Inc., New York, 1949), Seiten 199—200, verwiesen.

Das Verfahren der US-PS 1 716 082 führt zwar zu OTS-Salzen, die sich durch Aminierung in TS überführen lassen, doch betragen die Ausbeuten an OTS-Salzen lediglich 85 bis 90% der Theorie, und es werden hierzu Umsetzungszeiten von 4 bis 6 Stunden benötigt, was schlechte Raum-Zeit-Ausbeuten bedingt.

Aus dem britischen Patent 436 464 ist ein Verfahren zur Herstellung von Naphthylaminosulfonsäuren, wie Tobias-Säure, Brönner-Säure oder auch der Badischen-Säure bekannt. In dieser Patentschrift wird auf die Vorteile der Eintopfreaktion hingewiesen, wonach beispielsweise Tobias-Säure ausgehend von $\beta$-Naphthol hergestellt wird, ohne die dabei als Zwischenprodukt anfallende Oxy-Tobias-Säure zu isolieren. Das Reaktionsgemisch aus der Sulfonierung wird vielmehr neutralisiert, was mit wäßrigem Ammoniak, Natriumcarbonat, Natronlauge oder anderen basisch wirkenden Verbindungen geschehen kann, wobei die Temperatur jedoch nicht über 40°C steigen sollte und dann in Form der wäßrigen Reaktionslösung der Aminierung (Bucherer-Reaktion) unterworfen.

In US-PS 2 058 911 wird ein Verfahren zur Herstellung von TS beschrieben, das darin besteht, daß man die bei einer entsprechenden Aminierungsreaktion erhaltene Produktlösung mit einem mit Wasser nicht mischbaren organischen Lösungsmittel, wie Monochlorbenzol oder Dichlorbenzol, von gebildeten AN extrahiert und die hierdurch anfallende gereinigte wäßrige Lösung von TS-Natriumsalz dann bei einer Temperatur von etwa 45°C mit Chlorwasserstoffsäure bis auf Kongorot (pH 3,0) ansäuert, wodurch man zu einer TS gelangt, die sich als Zwischenprodukt zur Herstellung von Pigmentfarben eignet. Diese Operation bedingt erheblichen Aufwand und Ausbeuteverluste.

Des weiteren befassen sich die DE-OS 2 831 956, 2 831 965, 2 831 966, 2 831 992, 2 831 993, 2 831 994 und 2 831 995 mit der Herstellung 2-Naphthylamin-armer TS. Bei diesen Verfahren wird die Lösung der Aufgabe, der Herstellung einer 2-Naphthylamin-armen TS darin gesucht, daß man das angeblich durch Zersetzung von OTS- oder TS-Salzen entstehende 2-Naphthylamin zu verhindern sucht.

Da man auch in diesem Falle in der gesamten Verfahrenskonzeption bei den geringer löslichen Alkalisalzen der OTS blieb, konnte eine befriedigende Gesamtlösung, insbesondere in bezug auf die großtechnisch so wichtige Raum-Zeit-Ausbeute noch nicht erzielt werden.

Die Erfindung hat sich daher die Aufgabe gestellt, ein neues Verfahren zur Herstellung von 2-Amino-1-naphthalinsulfonsäure zu entwickeln, das die Nachteile der bekannten Verfahren nicht besitzt.

Gelöst wird diese Aufgabe erfindungsgemäß durch ein verbessertes Verfahren zur Herstellung von 2-Amino-1-naphthalinsulfonsäure (Tobias-Säure) (TS), bei dem man eine Suspension von 2-Hydroxynaphthalin (HN) in einem mit Wasser nicht mischbaren organischen Lösungsmittel mit Chlorsulfonsäure unter wasserfreien Bedingungen bei einer Temperatur von etwa 0°C bis 10°C zu 2-Hydroxy-1-naphthalinsulfonsäure (OTS) umsetzt, das entstandene Reaktionsgemisch einer Vakuum-HCl-Desorption unterwirft und durch Zusatz wäßriger Ammoniaklösung und Wasser in ein 2-Phasen-Gemisch, bestehend aus einer organischen und einer wäßrigen Phase überführt, die erhaltene ammoniakalisch-wäßrige Phase abtrennt, diese mittels einem mit Wasser nicht mischbaren organischen Lösungsmittel zwecks Entfernung von 2-Naphthol extrahiert und das restliche org. Lösungsmittel in der wäßrigen Phase im Vakuum abstrippt, die extrahierte wäßrige Schicht unter Druck der Einwirkung von Ammoniak und $SO_2$ abgebenden Mitteln aussetzt und erhitzt, das dadurch gebildete TS-Ammoniumsalz enthaltende wäßrige Reaktionsgemisch einem $NH_3$-Stripping unterwirft und mit einem mit Wasser nicht mischbaren organischen Lösungsmittel extrahiert, die extrahierte wäßrige Aufschlämmung von TS in der Wärme ansäuert, die Suspension einer $SO_2$-Desorption unterwirft, auf ca. 30°C abkühlt und das erhaltene Produkt abfiltriert und gegebenenfalls trocknet, das dadurch gekennzeichnet ist, daß man

a)   in ein geeignetes Reaktionsgefäß gleichzeitig und kontinuierlich getrennte Reaktantenströme aus einer 20- bis 30%igen Suspension von 2-Hydroxy-naphthalin in einem mit Wasser nicht mischbaren organischen gegen Chlorsulfonsäure inerten Lösungsmittel und aus gegebenenfalls mit dem gleichen Lösungsmittel verdünnter Chlorsulfonsäure bei einer Temperatur von −10°C bis 10°C mit derartiger Konzentration und Zugabegeschwindigkeit einführt, daß sich ein wasserfreies Reaktionsgemisch mit einem Molverhältnis von Chlorsulfonsäure zu 2-Hydroxy-naphthalin von 0,95 bis 1,00 zu 1,00 ergibt, und das Reaktionsgemisch etwa 10 bis 150 Minuten in diesem Reaktionsgefäß läßt,

b)   das in obiger Weise erhaltene Reaktionsgemisch unter Vakuum von 100−400 mbar bei 0−60°C, vorzugsweise 5−15°C, einer HCl-Desorption unterwirft,

c)   das verbleibende Reaktionsgemisch während 10−60 Minuten mit einer wäßrigen, 25- bis 35%igen Ammoniaklösung bei 60-75°C behandelt, bis der pH-Wert der vorher sauren Lösung mindestens 6 beträgt und mit soviel weiterem Wasser versetzt, daß sich deutlich zwei Flüssigphasen bilden, vorteilhaft mit der mindestens gleichen Menge Wasser wie der vorher zugesetzten Ammoniaklösung, wonach man

d)   die organische Lösungsmittelphase abtrennt und die erhaltene wäßrige Phase nach Extraktion der HN-Reste mittels einem organischen mit Wasser nicht mischbaren, vorzugsweise dem unter a) verwendeten Lösungsmittel, die Lösungsmittelreste mittels Vakuum von 200−400 mbar bei 60−80°C abstrippt,

e)   die verbliebene OTS-Ammoniumsalzlösung ohne vorherige Aufkonzentrierung mittels $NH_3$ und konzentrierter $(NH_4)_2SO_3$- oder $NH_4HSO_3$-Lösung bei 120−150°C, bei 10 bis 20 bar Druck während 4−12 Std. bis zu einem pH-Wert von 9−12 behandelt und nach erfolgtem anschließenden $NH_3$-Stripping nach gegebenenfalls vorheriger Umwandlung des Ammoniumsalzes mittels Zugabe von 20- bis 40%iger Alkalilösung bei 50−80°C in das Alkalisalz, und 2-Naphthylamin-Extraktion mittels eines Kohlenwasserstoffs,

f)   dem erhaltenen Reaktiongsgemisch bei 30−70°C soviel verdünnte 40- bis 60%ige Schwefelsäure zusetzt, daß ein pH-Wert von 1,2−1,75 resultiert, die erhaltene Suspension einer $SO_2$-Desorption im Vakuum bei 0,1−0,9 bar und 30−70°C, vorteilhaft 0,1−0,5 bar und 45−55°C, unterwirft und nach Abkühlen auf ca. 30°C die erhaltene reine TS durch Filtration abtrennt und gegebenenfalls trocknet.

Aus ökologischen Gründen ist es vorteilhaft, das Ammoniumsalz der erhaltenen TS vor der Ausfällung mittels Schwefelsäure mit Alkali zu behandeln. Dadurch kann der größte Teil des in die Reaktion eingeführten Ammoniaks zurückgewonnen werden, das somit für ein Reaktionsrecycling wieder zur Verfügung steht.

Folgende Tabelle der Löslichkeitswerte der Na- bzw. $NH_4$-Salze der Oxy-Tobias-Säure gibt indizierenden Aufschluß über die Möglichkeit der erzielbaren besseren Raum-Zeit-Ausbeuten des neuen Verfahrens.

Löslichkeit von Oxy-Tobias-Säure als Na-Salz
und NH₄-Salz in Wasser
in Abhängigkeit von der Temperatur

Tabelle 1

| Temp. °C | Na-Salz Gew.-% freie Säure | NH-Salz Gew.-% freie Säure |
|---|---|---|
| 20 | 32,5 | 38,0 |
| 30 | 34,0 | 41,5 |
| 35 | 35,5 | 43,0 |
| 45 | 38,0 | 47,0 |
| 55 | 41,5 | 51,5 |

Geeignete mit Wasser nicht mischbare, gegen Chlorsulfonsäure inerte, organische Lösungsmittel als Reaktionsmedien für das vorliegende Verfahren sind chlorierte Kohlenwasserstoffe, wie o-Dichlorbenzol, Monochlorbenzol, insbesondere aber Dichloräthan, ferner Dichlormethan bzw. Tetrachloräthan. Als Extraktionsmittel in den Stufen d) und e) kommen aromatische Kohlenwasserstoffe wie Toluol, Xylole bzw. Äthylbenzol sowie die gleichen wie vorher genannten in Frage.

Hervorzuheben ist als besonderer Vorteil des neuen Verfahrens, daß die Reaktion nicht aus in Lösung befindlichen Substraten gestartet werden muß, sondern bereits aus Suspensionen.

Da die Reaktion mit ca. 25%iger Suspension von HN gestartet werden kann, hat dies zum Effekt, daß ein um ca. 60% höherer Einsatz in die Reaktion möglich ist. Aufgrund kürzerer Reaktionszeiten in den Folgestufen und besserer Löslichkeit der Ammoniumsalze ermöglicht das neue Verfahren eine um ca. 100% bessere Raum-Zeit-Ausbeute gegenüber bisher bekannten Verfahren.

Für das vorliegende Verfahren wird reine oder Recycling-Chlor-Sulfonsäure in Lösung bzw. Mischung des gleichen Lösungsmittels bzw. Suspensionsmittels wie für die HN in Konzentration von ca. 50 Gew.-% eingesetzt. Niedrigere Konzentrationen sind wegen Raum-Zeit-Ausbeuten-Verlust nicht angebracht. Höhere können im Einzelfall angewandt werden.

Vorteilhafterweise wird Chlorsulfonsäure verwendet, die durch Verwertung von Chlorwasserstoff, das bei der Sulfonierung bzw. Sulfochlorierung mit Chlorsulfonsäure, beispielsweise von aromatischen Kohlenwasserstoffen, wie Naphthalin, Anthracen oder Benzol, in einem inerten organischen Lösungsmittel als Reaktionsmedium anfällt.

Ein solches Chlorwasserstoffgas müßte normalerweise, da es in Abhängigkeit vom unter den gegebenen Reaktionsbedingungen vorherrschenden Dampfdruck des verwendeten organischen Lösungsmittels mehr oder weniger stark mit letzterem beladen ist, unter großem Aufwand vernichtet werden.

Denn um dieses verunreinigte Chlorwasserstoffgas wieder verwerten zu können, müßte es zuerst vom Lösungsmittel befreit werden, was aufwendige Operationen in korrosionsfreien Apparaturen erfordern würde.

Dieses mit Lösungsmittel beladene Chlorwasserstoffgas wird mit Schwefeltrioxid bei Temperaturen unterhalb 60°C in stöchiometrischen Mengen oder im geringen Überschuß von Chlorwasserstoffgas in Chlorsulfonsäure als Reaktionsmedium zu Chlorsulfonsäure umgesetzt.

Da die dem Chlorwasserstoffgas anhaftenden inerten Lösungsmittel den ganzen Chlorsulfonsäure-Herstellprozeß durchlaufen, ist die erhaltene Chlorsulfonsäure lösungsmittelhaltig. Dennoch stellt diese ein wertvolles chemisches Reagens dar, das in Sulfonierungs- bzw. Sulfochlorierungsverfahren, die in gleichem oder ähnlichem Lösungsmittelmedium ablaufen, wie z. B. im vorliegenden Verfahren, ohne weiteres verwendet werden kann.

Weitere zur Anwendung kommende Reagenzien sind Ammoniak bzw. Ammoniak und Schwefeldioxid liefernde Verbindungen wie z. B. wäßrige 25- bis 35%ige Ammoniaklösung oder Ammoniumbisulfit-Lösung.

Das neue Verfahren wird vorzugsweise kontinuierlich durchgeführt. Obwohl die einzelnen Stufen batch-weise ablaufen, ist eine kontinuierliche Reaktionsführung dadurch erreicht worden, daß man entsprechend dimensionierte Stapelgefäße als Puffer in den Gesamtprozeß eingebaut hat.

Im einzelnen kann das vorliegende Verfahren beispielsweise folgendermaßen durchgeführt werden, wie es mit anliegender Skizze erläutert wird.

Die Reaktanden werden in Vorratsgefäßen angerichtet, HN in 20- bis 30%iger Suspension in einem organischen Lösungsmittel, das gegen Chlorsulfonsäure inert ist sowie Chlorsulfonsäure, gegebenenfalls mit dem gleichen organischen Lösungsmittel wie dem, das als Suspensionsmedium für HN dient, verdünnt.

Aus beiden Vorratsgefäßen werden die Reaktanden nun synchron in eine Rührgefäßkaskade I dosiert. Die Dosiergeschwindigkeit richtet sich nach der Dimensionierung der Apparatur und der Kühlleistung der Kühlaggregate, da die Reaktion exotherm ist und eine Reaktionstemperatur von 0–10°C eingehalten werden soll.

Die Synchronisierung der Dosierung der Reaktanden muß so aufeinander abgestimmt sein, daß sich ein Molverhältnis von Chlorsulfonsäure zu HN von 0,95 bis 1,00 zu 1,00 ergibt. Die Verweilzeit in dieser Kaskade hängt von den obengenannten Bedingungen ab. Im Mittel beträgt sie ca. 70 Minuten.

Das entstehende Chlorwasserstoffgas wird abgesaugt und absorbiert bzw. zwecks erneuter Chlorsulfonsäureherstellung, wie vorher beschrieben, recycliert.

Das im Reaktionsmedium gelöste restliche Chlorwasserstoffgas wird unter Vacuum von 100—400 mbar und bei 0—60°C desorbiert. Vorteilhaft arbeitet man jedoch im Bereich von 5—15°C, insbesondere 5—10°C.

Die erhaltene Sulfiermasse wird nun simultan mit 25- bis 35%iger wäßriger Ammoniaklösung dem Neutralisations- und Reaktionsgefäß II zugeführt, wobei die Dosiergeschwindigkeit so eingestellt wird, daß ein pH-Wert von 6—6,5 resultiert. Die Temperatur beträgt 60—75°C.

Gleichzeitig wird der Reaktionsmasse noch soviel Wasser zugesetzt, daß sich zwei Flüssigphasen bilden. Es bilden sich eine wäßrige Produktphase und die des als Reaktionsmedium dienenden org. Lösungsmittels. Die Wassermenge hängt jedoch primär von der Konzentration ab, in der man im weiteren Verlauf zu arbeiten wünscht. Aus ökonomischen Gründen wird die Konzentration der Produktmasse so hoch wie möglich, d. h. dicht unter der Löslichkeitsgrenze der OTS gehalten — siehe hierzu Tabelle 1. Man setzt vorteilhaft mindestens gleich viel Wasser zu, wie zuvor Ammoniaklösung.

Die Verweilzeit beträgt im Gefäß II zwischen 10—60 Minuten.

Das Zweiphasengemisch wid nun in Trenngefäß III geleitet, und bei der gleichen Temperatur wie in Gefäß II wird die org. Lösungsmittelphase von der wäßrigen Produktphase getrennt.

Die erhaltene wäßrige Phase wird in einer Gegenstromextraktionskolonne IV von nicht umgesetzten HN-Resten unter Verwendung des gleichen Lösungsmittels, wie eingangs als Reaktionsmedium benutzt, befreit. Die Temperatur beträgt hier 40—80°C. Das in der wäßrigen Phase verbleibende Lösungsmittel wird nach der Extraktion im Stripper V unter Vakuum von 200—400 mbar und einer Temperatur von 60—80°C abdestilliert.

Die wäßrige Phase enthält nun das Ammoniumsalz der Oxytobiassäure als Zwischenprodukt.

Bei einer Temperatur von ca. 70'C liegt die Konzentration des gelösten Salzes im Bereich zwischen 50—55%. Diese hochkonzentrierte Arbeitsweise führt zu sehr guten Ergebnissen in bezug auf Raum-Zeit-Ausbeute und Produktqualität.

Die OTS-NH$_4$-Salz-Lösung wird nun in eine Reaktionskolonne VI eingeleitet. Gleichzeitig wird flüssiges Ammoniak und 40- bis 55%ige NH$_4$HSO$_3$-Lösung in molaren Mengen bis zu 50% Überschuß, bezogen auf das OTS-Salz, in Reaktor VI eingeleitet. Die Temperatur wird auf 130—150°C erhöht, wodurch sich ein Druck von 10—20 bar einstellt. Unter diesen Bedingungen beträgt die Verweilzeit im Reaktor 4—12 Std., im Mittel 8—10 Std., der pH-Wert vorteilhaft 9—11.

Danach wird die Reaktionsmasse entspannt und mit soviel 20- bis 40%iger, wäßriger NaOH-Lösung versetzt, daß alles auf diese Art freizusetzende Ammoniak entweicht.

In Kolonne VII wird das restliche Ammoniak abgestrippt. Das erhaltene TS-Na-Salz wird nun mit Wasser auf eine Konzentration von 20—30% eingestellt und in Gegenstromextraktionskolonne VIII mittels Toluol oder ähnlichen aromatischen Kohlenwasserstoffen bei ca. 60—80°C extrahiert, um die noch vorhandenen kleinen Mengen AN zu entfernen.

Danach resultiert ein TS-Na-Salz mit weniger als 30 ppm AN.

Im Kessel IX erfolgt nun durch jeweils simultanes Zudosieren von 40- bis 60%iger Schwefelsäure und Reaktionsgemisch bei einer Temperatur von 40—70°C, vorzugsweise 45—55°C, die pH-kontrollierte Ausfällung der Tobiassäure. Dabei wird soviel Säure zugesetzt, daß ein pH-Wert von 1,2—1,75 vorzugsweise 1,5—1,75 resultiert.

Diese Operation dauert 20—40 Minuten. Das bei dieser Handlung freigesetzte SO$_2$ kann sofort aus diesem Kessel oder alternativ aus der Desorptionskolonne X abgesaugt oder mit einem Inertgas ausgetrieben werden, und in den Prozeß zur Herstellung von Ammoniumbisulfitlösung zurückgeführt werden. Die erhaltene Tobiassäure wird in XI mittels Filterpresse abfiltriert, mit Wasser neutral gewaschen und im Vakuum getrocknet.

Die Ausbeute an TS liegt nach dem erfindungsgemäßen Verfahren, bezogen auf eingesetztes HN, zwischen 93—98%.

Die gemäß dem neuen Verfahren hergestellte Tobiassäure stellt ein wertvolles Zwischenprodukt zur Herstellung von Farbstoffen dar. Insbesondere dient es als Diazo- oder Kupplungskomponente bei der Herstellung von Diazofarbstoffen.

Das neue Verfahren kann gemäß nachfolgendem Beispiel durchgeführt werden. Es bestehen natürlich in Abhängigkeit von den angewandten Konzentrationen der verschiedenen Reaktanden Variationsmöglichkeiten.

### Beispiel

Eine 22,6%ige kristalline, gut fließbare Suspension von $\beta$-Naphthol in 1,2-Dichloräthan wird mit einer Dosiergeschwindigkeit von 1560 g/h (=2,44 Mol/h) synchron mit 279 g/h (=2,39 Mol/h) Chlorsulfonsäure in eine Rührgefäßkaskade dosiert. Die Reaktionstemperatur wird bei 0 bis 5°C gehalten, die Verweilzeit beträgt ca. 75 Minuten. Der bei der Sulfierung gebildete Chlorwasserstoff wird absorbiert, und der in der Reaktionsmasse noch gelöste Chlorwasserstoff wird durch eine Vakuumdesorption bei 0 bis 5°C entfernt.

Die Sulfiermasse wird nun kontinuierlich mit 145 g/h (=2,55 Mol/h) einer 30%igen Ammoniaklösung in einem Rührgefäß bis pH 6—6,5 und 70—75°C versetzt, gleichzeitig mit 234 g/h Wasser verdünnt und nach einer Verweilzeit von 22 Minuten in einen beheizten Abscheider gepumpt und von der wäßrigen Produktphase bei 70 bis 75°C getrennt.

Die gewonnene, wäßrige Phase (Oxy-Tobiassäure-Ammoniumsalzlösung) wid in einer mehrstufigen beheizten Gegenstromextraktionskolonne mit 1000 g/h 1,2-Dichloräthan bei 70 bis 75°C von nicht umgesetztem β-Naphthol befreit.

Das danach in der Oxy-Tobiassäure-Ammoniumsalzlösung noch gelöste 1,2-Dichloräthan wird durch Destillation bei etwa 390 mbar und 70 bis 75°C entfernt.

Die β-naphtholhaltige 1,2-Dichloräthan-Phase wird mit Wasser extrahiert, entwässert und in den Kreislauf zurückgeführt.

Es resultieren 1100 g/h einer 51,4%igen Oxy-Tobiassäure-Ammoniumsalzlösung, die dichloräthanfrei ist und etwa 30 ppm β-Naphthol enthält. Die Oxy-Tobiassäure-Ausbeute beträgt 98,3% d. Th., bezogen auf β-Naphthol-Einsatz, bis zu dieser Reaktionsphase.

In eine mit Rührer versehene, in Kammern unterteilte Reaktionskolonne werden gleichzeitig 1100 g/h Oxy-Tobiassäure-Ammoniumsalzlösung (51,4%ig); 165 g/h Ammoniak 100% und 231 g/h Ammoniumbisulfitlösung 54%ig zugeführt. Die Temperatur im Reaktor wird bei 140°C gehalten. Die Verweilzeit beträgt 8,5 h. Der Druck wird auf 15 bar eingestellt.

Die Oxy-Tobiassäure wird unter diesen Bedingungen praktisch vollständig (>98%) zu TS umgesetzt.

Nach dem Passieren durch den Reaktor wird das Reaktionsgemisch auf Atmosphären-Druck entspannt und mit wäßriger, 20%iger NaOH-Lösung, 480 g/h, vermischt, um die Ammoniumsalze in die Natriumsalze umzuwandeln. Anschließend wird das NH$_3$ in einer Destillationskolonne ausgestrippt und in den Prozeß zurückgeführt. Die am Boden der Kolonne abfließende Produktlösung wird mit Wasser auf eine Konzentration von ca. 250 g TS-Na-Salz pro Liter eingestellt und in einer Gegenstrom-Extraktionskolonne mit Toluol kontinuierlich bei 70—75°C extrahiert. Auf diese Weise wird 2-Naphthylamin bis auf eine geringe Restmenge von ca. 10 ppm, bezüglich TS, aus der Produktlösung entfernt.

Aus dieser Lösung wird nun durch zudosieren von 50%iger Schwefelsäure, ca. 540 g/h, die freie TS ausgefällt. Die Temperatur wird bei 50—55°C gehalten und der pH-Wert zwischen 1,5—1,75. Das hierbei freigesetzte SO$_2$ wird desorbiert und in den Prozeß zurückgeführt.

Man filtriert, wäscht mit kaltem Wasser und trocknet das feuchte Produkt im Vakuum bei 70—75°C.

Die Ausbeute an isolierter TS beträgt 95% d. Th. bezogen auf Oxytobiassäure. Der 2-Naphthylamin-Gehalt beträgt <30 ppm.

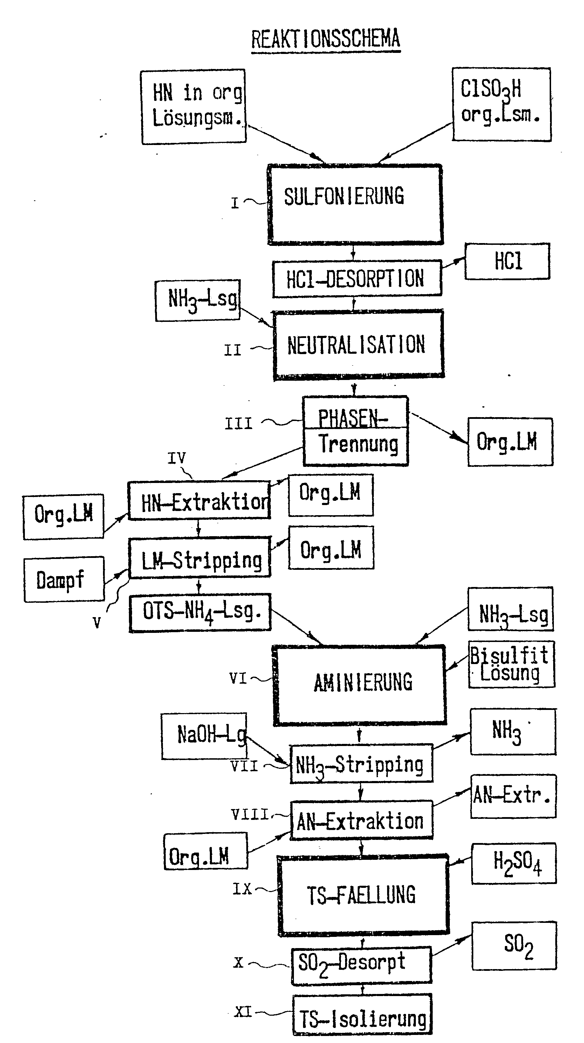

## Patentansprüche

1. Verfahren zur Herstellung von 2-Amino-1-naphthalinsulfonsäure (Tobias-Säure) (TS), indem man eine Suspension von 2-Hydroxynaphthalin (HN) in einem mit Wasser nicht mischbaren organischen Lösungsmittel mit Chlorsulfonsäure unter wasserfreien Bedingungen bei einer Temperatur von etwa 0°C bis 10°C zu 2-Hydroxy-1-naphthalinsulfonsäure (OTS) umsetzt, das entstandene Reaktionsgemisch einer Vakuum-HCl-Desorption unterwirft und durch Zusatz von wäßriger Ammoniaklösung und Wasser in ein 2-Phasen-Gemisch, bestehend aus einer organischen und einer wäßrigen Phase überführt, die erhaltene ammoniakalisch-wäßrige Phase abtrennt, diese mittels einem mit Wasser nicht mischbaren organischen Lösungsmittel zwecks Entfernung von 2-Naphthol extrahiert und das restliche org. Lösungsmittel in der wäßrigen Phase im Vakuum abstrippt, die extrahierte wäßrige Schicht unter Druck der Einwirkung von Ammoniak und SO$_2$ abgebenden Mitteln aussetzt und erhitzt, das dadurch gebildete TS-Ammoniumsalz enthaltende wäßrige Reaktionsgemisch einem NH$_3$-Stripping unterwirft und mit einem mit Wasser nicht mischbaren organischen Lösungsmittel extrahiert, die extrahierte wäßrige Lösung von TS-Salz in der Wärme ansäuert, die Suspension einer SO$_2$-Desorption unterwirft, auf ca. 30°C abkühlt und das erhaltene Produkt abfiltriert und gegebenenfalls trocknet, dadurch gekennzeichnet, daß man

a) in ein geeignetes Reaktionsgefäß gleichzeitig und kontinuierlich getrennte Reaktantenströme, bestehend aus einer 20- bis 30%igen Suspension von 2-Hydroxy-naphthalin in einem mit Wasser nicht mischbaren organischen, gegen Chlorsulfonsäure inerten Lösungsmittel und aus Chlorsulfonsäure bei einer Temperatur von −10°C bis 10°C mit derartiger Konzentration und Zugabegeschwindigkeit einführt, daß sich ein wasserfreies Reaktionsgemisch mit einem Molverhältnis von Chlorsulfonsäure zu 2-Hydroxy-naphthalin von 0,95 bis 1,00 zu 1,00 ergibt, und das Reaktionsgemisch etwa 10 bis 150 Minuten in diesem Reaktionsgefäß läßt,

b) das in obiger Weise erhaltene Reaktionsgemisch unter Vakuum von 100—400 mbar bei 0—60°C einer HCl-Desorption unterwirft,

c) das verbleibende Reaktionsgemisch während 10—60 Minuten mit einer wäßrigen, 25—35%igen Ammoniaklösung bei 60—75°C behandelt, bis der pH-Wert der vorher sauren Lösung mindestens 6 beträgt, und mit soviel weiterem Wasser versetzt, daß sich deutlich zwei Flüssigphasen bilden, wonach man

d) die organische Lösungsmittelphase abtrennt und die erhaltene wäßrige Phase nach Extraktion der 2-Hydroxy-Naphthalin-Reste mittels einem organischen mit Wasser nicht mischbaren Lösungsmittel, die Lösungsmittelreste mittels Vakuum von 200—400 mbar bei 60—80°C abstrippt,

e) die verbliebene OTS-Ammoniumsalzlösung ohne vorherige Aufkonzentrierung mittels NH$_3$ und konzentrierter (NH$_4$)$_2$SO$_3$- oder NH$_4$HSO$_3$-Lösung bei 120—150°C bei 10 bis 20 bar Druck während 4—12 Std. bis zu einem pH-Wert von 9—12 behandelt und nach erfolgtem anschließendem NH$_3$-Stripping

und 2-Naphthylamin-Extraktion mittels eines Kohlenwasserstoffs,

f) dem erhaltenen Reaktionsgemisch bei 30—70°C soviel verdünnte 40- bis 60%ige Schwefelsäure zusetzt, daß ein pH-Wert von 1,2—1,75 resultiert, die erhaltene Suspension einer SO₂-Desorption im Vakuum bei 0,1—0,9 bar und 30—70°C unterwirft und nach Abkühlen auf ca. 30°C die erhaltene reine TS durch Filtration abtrennt, mit Wasser wäscht und falls erforderlich trocknet.

2. Verfahren gemäß Anspruch 1, dadurch gekennzeichnet, daß in Stufe a) die Chlorsulfonsäure mit dem gleichen Lösungsmittel verdünnt, das auch für die Extraktion des 2-Hydroxynaphthalins verwendet wird, die HCl-Desorption in Stufe b) bei 5—15°C durchführt, in Stufe c) nach der Ammoniakzugabe noch mindestens die gleiche Menge Wasser, wie der vorher zugesetzten Ammoniaklösung zwecks besserer Phasentrennung zusetzt, in Stufe d) mit dem gleichen Lösungsmittel extrahiert, wie es in Stufe a) eingesetzt wird, und in Stufe e) vor dem NH₃-Stripping oder Abtreiben das Ammoniumsalz der TS zwecks Rückgewinnung des Ammoniaks durch Zugabe von 20—40% wäßriger Alkali-Lösung bei 50—80°C in das Alkalisalz umwandelt.

3. Verfahren gemäß Anspruch 1, dadurch gekennzeichnet, daß man als mit Wasser nicht mischbare, gegen Chlorsulfonsäure inerte organische Lösungsmittel, Monochlorbenzol, Dichlorbenzol, Dichloräthan, Dichlormethan bzw. Tetrachloräthan einsetzt.

4. Verfahren gemäß Anspruch 1, dadurch gekennzeichnet, daß man in den Stufen d) und e) als Extraktionsmittel aromatische Kohlenwasserstoffe wie Toluol, Xylol oder Äthylbenzol verwendet.

5. Verfahren gemäß Anspruch 1, dadurch gekennzeichnet, daß man in Stufe a) in eine Rührgefäßkaskade gleichzeitig und kontinuierlich getrennte Reaktantenströme aus einer 23%igen Suspension von 2-Hydroxy-naphthalin in 1,2-Dichloräthan und aus mit dem gleichen Lösungsmittel verdünnter Chlorsulfonsäure bei einer Temperatur von 0°C bis 5°C mit derartiger Konzentration und Zugabegeschwindigkeit einleitet, daß sich ein wasserfreies Reaktionsgemisch mit einem Molverhältnis von Chlorsulfonsäure zu 2-Hydroxy-naphthalin von 0,95 zu 1,00 ergibt, und das Reaktionsgemisch etwa 70 Minuten in diesem Reaktionsgefäß läßt,

in Stufe b)
das in obiger Weise erhaltene Reaktionsgemisch unter Vakuum von 100—400 mbar bei 5—10°C einer HCl-Desorption unterwirft,

in Stufe c)
das verbleibende Reaktionsgemisch während 10—60 Minuten mit einer wäßrigen, 25- bis 35%igen Ammoniaklösung bei 60—75°C behandelt, bis der pH-Wert der vorher sauren Lösung mindestens 6 beträgt und mit der mindestens gleichen Menge Wasser wie der vorher zugesetzten Ammoniaklösung versetzt, wonach man

in Stufe d)
die organische Lösungsmittelphase abtrennt und die erhaltene wäßrige Phase nach Extraktion der NH-Reste mittels 1,2-Dichloräthan, aus dieser Phase die Lösungsmittelreste mittels Vakuum von 200—400 bar bei 60—80°C abstrippt,

in Stufe e)
das verbliebene OTS-Ammoniumsalz ohne vorherige Aufkonzentrierung mittels flüssigem NH₃- und konzentrierter NH₄HSO₃-Lösung bei 120—150°C, bei 10 bis 20 bar Druck während 8—10 Std. bis zu einem pH-Wert von 9—11 behandelt und nach erfolgtem anschließendem NH₃-Stripping nach vorheriger Umwandlung des Ammoniumsalzes mittels Zugabe von 20- bis 40%iger Alkalilösung bei 50—80°C in das Alkalisalz und 2-Naphthylamin-Extraktion mittels Toluol,

in Stufe f)
das erhaltene Reaktionsgemisch bei 45—55°C mit soviel verdünnter 40- bis 60%iger Schwefelsäure simultan zusammenführt, daß ein pH-Wert von zwischen 1,5 bis 1,75 resultiert, die erhaltene Suspension jeweils einer SO₂-Desorption im Vakuum bei 0,1—0,5 bar und 45—55 unterwirft resp. einem Inertgas bei Normaldruck austreibt und nach Abkühlen auf ca. 30°C die erhaltene reine TS durch Filtration abtrennt, mit Wasser wäscht und trocknet.

**Claims**

1. A process for the production of 2-amino-1-naphthalenesulfonic acid (tobias acid) by reacting a suspension of 2-hydroxynaphthalene in a water-immiscible organic solvent with chlorosulfonic acid, under anhydrous conditions and in the temperature range from about 0°C to 10°C, to give 2-hydroxy-1-naphthalenesulfonic acid (oxy-tobias acid), removing HCl from the reaction mixture by desorption in vacuo and, by addition of aqueous ammonia solution and water, converting the reaction mixture into a two-phase mixture consisting of an organic and an aqueous phase, separating the resultant ammoniacal-aqueous phase and extracting it with a water-immiscible organic solvent to remove 2-naphthol and stripping off the residual organic solvent in the aqueous phase in vacuo, treating the extracted aqueous layer, under pressure, with ammonia and SO₂ donors and heating it, stripping off NH₃ from the aqueous reaction mixture containing the ammonium salt of tobias acid thereby obtained and extracting the product solution with a water-immiscible organic solvent, acidifying the extracted aqueous suspension of Tobias acid salt at elevated temperature, removing $SO_2$ from the suspension by desorption, cooling to 30°C, and collecting the product by filtration, and, if desired, drying it, which process comprises

a) introducing into a suitable reaction vessel, simultaneously and continuously, spearate streams of a 20—30% suspension of 2-hydroxynaphthalene in a water-immiscible organic solvent which is inert to chlorosulfonic acid, and of chlorosulfonic acid, in the temperature range from −10° to +10°C, in such a concentration and at such a rate of addition that an anhydrous reaction mixture having a molar ratio of chlorosulfonic acid to 2-hydroxynaphthalene of about 0.95 to 1.00 to 1 is obtained, and leaving the reaction mixture for about 10 to 150 minutes in the reaction vessel,

b) removing HCl from the reaction mixture obtained as described above by desorption in vacuo under a pressure of 100—400 mbar in the temperature range from 0° to 60°C,

c) treating the residual reaction mixture for 10 to 60 minutes with an aqueous 25—35% ammonia solution in the temperature range from 60° to 75°C until the pH value of the previously acid solution is at least 6 and adding sufficient further water that two liquid phases clearly form, subsequently

d) separating the organic solvent phase and the aqueous phase after removal of residual 2-hydroxynaphthalene by extraction with an organic water-immiscible solvent, stripping off remaining solvent in vacuo at 200—400 mbar and in the temperature range from 60° to 80°C,

e) treating the resultant ammonium salt of oxytobias acid, without increasing the concentration beforehand, with $NH_3$ an concentrated $(NH_4)_2SO_3$ or $NH_3HSO_3$ solution in the temperature range from 120° to 150°C and under a pressure of 10 to 20 mbar for 4 to 12 hours until the pH value is 9 to 12, and, after stripping off $NH_3$ and removal of 2-naphthylamine by extraction with an aromatic hydrocarbon,

f) adding to the reaction mixture, in the temperature range from 30° to 70°C, sufficient dilute 40—60% sulfuric acid to adjust the pH value to 1.2—1.75, removing $SO_2$ from the resultant suspension by desorption in vacuo at 0.1 to 0.9 bar and in the temperature range form 30° to 70°C, and, after cooling to about 30°C, isolating the pure tobias acid by filtration, washing it with water and, if necessary, drying it.

2. A process according to claim 1, which comprises diluting the chlorosulfonic acid in step a) with the same solvent as is used for the extraction of 2-hydroxynaphthalene, carrying out the desorption of HCl in step b) in the temperature range from 5° to 15°C, in step c), after the addition of ammonia, adding at least the same amount of water as of the previously added ammonia for better phase separation, extracting the aqueous phase in step d) with the same solvent as is used in step a), and, in step e), before stripping off the ammonia, converting the ammonium salt of tobias acid into the alkali metal salt to recover the ammonia by addition of 20—40% aqueous alkali solution, in the temperature range from 50° to 80°C.

3. A process according to claim 1, wherein the waterimmiscible organic solvent which is inert to chlorosulfonic acid is monochlorobenzene, dichlorobenzene, dichloroethane or tetrachloroethane.

4. A process according to claim 1, wherein the solvent used for extraction in steps d) and e) is an aromatic hydrocarbon such as toluene, xylene or ethyl benzene.

5. A process according to claim 1 which comprises, in step a), introducing into an agitator vessel cascade, simultaneously and continuously, separate streams of reactants consisting of a 23% suspension of 2-hydroxynaphthalene in 1,2-dichloroethane and of chlorosulfonic acid diluted wiht the same solvent, in the temperature range from 0° to 5°C, in such a concentration and at such a rate of addition that an anhydrous reaction mixture with a molar ratio of chlorosulfonic acid to 2-hydroxynaphthalene of about 0.95 to 1 is obtained, and leaving the reaction mixture for about 70 minutes in the reaction vessel, in step b) removing hydrogen chloride from the reaction mixture so obtained by desorption under a pressure of 100—400 mbar and in the temperature range from 5° to 10°C, in step c) adding a 23—35% ammonia solution to the remaining reaction mixture over the course of 10 to 60 minutes, and in the temperature range from 60° to 75°C, until the pH of the previously acid solution is at least 6, and diluting the reaction mixture with at least the same amount of water as of the previously added ammonia solution, in step d) separating the organic solvent phase and the resultant aqueous phase after removal of the remaining ammonia by extraction with 1,2-dichloroethane, and stripping off remaining solvent from this phase under a pressure of 200—400 mbar in the temperature range from 60° to 80°C, in step e) treating the residual ammonium salt of oxy-tobias acid, without increasing the concentration beforehand, with liquid $NH_3$ and concentrated $NH_4HSO_3$ solution in the temperature range from 120° to 150°C and under a pressure of 10 to 20 bar over the course of 8 to 10 hours until the pH value is 9—11, and, after stripping off $NH_3$ following conversion of the ammonium salt into the alkali metal salt by addition of 20—40% alkali solution in the temperature range from 50° to 80°C, and removing 2-naphthylamine by extraction with toluene, in step f) simultaneously adding resultant reaction mixture, in the temperature range from 45° to 55°C, to sufficient dilute 40—60% sulfuric acid that a pH value of 1.5 to 1.75 results, removing $SO_2$ from the resultant suspension by desorption in vacuo under a pressure of 0.1 to 0.5 bar and in the temperature range from 45° to 55°C or expelling it with an inert gas under normal pressure, and, after cooling to about 30°C, isolating the pure tobias acid by filtration, washing it with water and drying it.

## Revendications

1. Procédé de préparation d'acide 2-amino-1-naphtalène-sulfonique (acide de Tobias) (TS), réalisé en faisant réagir une suspension de 2-hydroxynaphtalène (HN), dans un solvant organique non miscible avec l'eau, avec la chlorhydrine sulfurique, dans des conditions anhydres, à une température d'environ 0°C à 10°C pour former de l'acide 2-hydroxy-1-naphtalène sulfonique (OTS), en soumettant le mélange réactionnel obtenu à une désorption, sous vide, de HCl, en le transformant en un mélange à deux phases constituées d'une phase organique et d'une phase aqueuse, par addition d'eau et d'une solution aqueuse d'ammoniac, en séparant la phase aqueuse ammoniacale obtenue, en extrayant celle-ci par un solvant organique non miscible avec l'eau pour éliminer le 2-naphtol et en chassant sous vide le reste de solvant organique contenu dans la phase aqueuse, en exposant sous pression la couche aqueuse extraite à l'action de l'ammoniac et d'agents donneurs de $SO_2$, et en chauffant, en soumettant le mélange réactionnel aqueux contenant le sel d'ammonium de TS ainsi formé à une élimination de $NH_3$ et en extrayant par un solvant organique non miscible avec l'eau, en acidifiant la solution aqueuse de sel de TS extraite à chaud, en soumettant la suspension à une désorption de $SO_2$, en la refroidissant à environ 30°C et en filtrant le produit obtenu et le séchant le cas échéant, caractérisé en ce que

a) on introduit simultanément et de façon continue dans un réacteur approprié des courants séparés de réactifs constitués par une suspension à 20—30% de 2-hydroxynaphtalène dans un solvant organique non miscible avec l'eau et inerte vis-à-vis de la chlorhydrine sulfurique et par la chlorhydrine sulfurique, à une température de −10°C à 10°C, à une concentration et à un débit tels que cela donne un mélange réactionnel anhydre avec un rapport moléculaire de chlorhydrine sulfurique au 2-hydroxynaphtalène de 0,95—1,00 pour 1, et on laisse le mélange réactionnel environ 10 à 150 minutes dans ce réacteur;

b) on soumet le mélange réactionnel obtenu de la manière décrite ci-dessus, sous vide de 100—400 mbars, à une désorption de HCl, à 0—60°C;

c) on traite le mélange réactionnel restant, pendant 10—60 minutes, avec une solution aqueuse d'ammoniaque à 25—35%, à 60—75°C, jusqu'à ce que le pH de la solution auparavant acide atteigne au moins 6 et on ajoute encore suffisamment d'eau, pour obtenir la formation de deux phases liquides distinctes, après quoi

d) on sépare la phase de solvant organique et on chasse de la phase aqueuse obtenue après extraction des restes de 2-hydroxynaphtalène au moyen d'un solvant organique non miscible avec l'eau, les restes de solvant, au moyen d'un vide de 200—400 mbars à 60—80°C;

e) on traite la solution restante de sel d'ammonium d'OTS sans concentration préalable, par $NH_3$ et une solution concentrée de $(NH_4)_2SO_3$- ou de $NH_4HSO_3$-, a 120—150°C, sous une pression de 10 à 20 bars, pendant 4—12 heures, jusqu'à un pH de 9—12; et après l'entraînement consécutif de $NH_3$, et l'extraction de la 2-naphtylamine, on traite au moyen d'un hydrocarbure;

f) on ajoute au mélange réactionnel obtenu à 30—70°C suffisamment d'acide sulfurique dilué à 40—60% pour obtenir un pH de 1,2—1,75, on soumet la suspension obtenue à une désorption de $SO_2$ sous vide de 0,1—0,9 bar et à 30—70°C et, après refroidissement à 30°C environ, on sépare le TS pur obtenu par filtration, on le lave à l'eau et on le sèche si nécessaire.

2. Procédé selon la revendication 1, caractérisé en ce que dans l'étape a) la chlorhydrine sulfurique est diluée avec le même solvant qui est utilisé aussi pour l'extraction du 2-hydroxynaphtalène, la désorption de HCl est réalisée dans l'étape b) à 5—15°C, dans l'étape c) après l'addition d'ammoniac on ajoute encore au moins la même quantité d'eau que celle de la solution d'ammoniac ajoutée auparavant afin d'obtenir une meilleure séparation de phases, dans l'étape d) on extrait avec le même solvant que celui utilisé dans l'étape a) et dans l'étape e) avant l'élimination ou l'entraînement de $NH_3$, on transforme le sel d'ammonium de TS en sel alcalin afin de récupérer l'ammoniac par addition d'une solution aqueuse d'alcali à 20—40%, à 50—80°C.

3. Procédé selon la revendication 1, caractérisé en ce qu'on utilise comme solvants organiques, non miscibles avec l'eau et inertes vis-à-vis de la chlorhydrine sulfurique, du monochlorobenzène, dichlorobenzène, dichloréthane, dichlorométhane ou tétrachloréthane.

4. Procédé selon la revendication 1, caractérisé en ce que dans les étapes d) et e) on utilise comme agent d'extraction un hydrocarbure aromatique comme le toluène, le xylène ou l'éthylbenzène.

5. Procédé selon la revendication 1, caractérisé en ce que dans l'étape a, on introduit simultanément et de façon continue dans une cascade de cuves munies d'un agitateur des courants séparés de réactifs d'une suspension à 23% de 2-hydroxynaphtalène dans le 1,2-dichloréthane et de chlorhydrine sulfurique diluée dans le même solvant à une température de 0°C à 5°C, à une concentration telle et à une vitesse d'addition telle, que cela donne un milieu réactionnel anhydre avec un rapport moléculaire de chlorhydrine sulfurique au 2-hydroxynaphtalène de 0,95 à 1,00 et on laisse le mélange réactionnel environ 70 minutes dans ce réacteur,

dans l'étape b
on soumet le mélange réactionnel obtenu de la manière décrite ci-dessus sous vide de 100—400 mbars à une désorption de HCl à 5—10°C,

dans l'étape c
on traite le mélange réactionnel restant pendant 10—60 minutes avec une solution aqueuse d'ammoniac à 25—35%, à 60—75°C, jusqu'à ce que le pH de la solution auparavant acide atteigne au moins 6 et on ajoute au moins la même quantité d'eau que celle de la solution ammoniacale ajoutée auparavant, après quoi

dans l'étape d
on sépare la phase de solvant organique et on chasse de la phase aqueuse obtenue après extraction du reste de HN au moyen de 1,2-dichloréthane, les restes de solvant au moyen d'un vide de 200—400 mbars, à 60—80°C,

dans l'étape e
on traite de sel d'ammonium d'OTS restant sans concentration préalable au moyen de $NH_3$ liquide et d'une solution concentrée de $NH_4HSO_3$, à 120—150°C, sous une pression de 10 à 20 bars, pendant 8—10 heures jusqu'à un pH de 9—11 et, après l'entraînement consécutif de $NH_3$ après la transformation préalable du sel d'ammonium en sel alcalin par addition d'une solution d'alcali à 20—40%, à 50—80°C, et extraction de la 2-naphtylamine au moyen du toluène,

dans l'étape f
on ajoute au mélange réactionnel obtenu à 45—55°C simultanément suffisamment d'acide sulfurique dilué à 40—60% pour obtenir un pH compris entre 1,5 et 1,75, on soumet la suspension obtenue à chaque fois à une désorption de $SO_2$ sous vide à 0,1—0,5 bar et 45—55°C ou à un entraînement par un gaz inerte à pression normale, et après refroidissement à 30°C environ, on sépare le TS pur obtenu par filtration, on le lave à l'eau et on le sèche.

## REAKTIONSSCHEMA

```
┌─────────────┐                              ┌─────────────┐
│ HN in org   │                              │ ClSO₃H      │
│ Lösungsm.   │                              │ org.Lsm.    │
└─────────────┘                              └─────────────┘
              ╲                              ╱
               ▼                            ▼
        ┌──────────────────────────┐
   I    │      SULFONIERUNG         │
        └──────────────────────────┘
                    │
                    ▼
        ┌──────────────────┐          ┌──────┐
        │  HCl-DESORPTION  │─────────▶│ HCl  │
        └──────────────────┘          └──────┘
   ┌─────────┐        │
   │ NH₃-Lsg │        ▼
   └─────────┘  ┌──────────────────┐
   II           │  NEUTRALISATION  │
                └──────────────────┘
                        │
                        ▼
              ┌──────────────┐         ┌────────┐
   III        │  PHASEN-     │────────▶│ Org.LM │
              │  Trennung    │         └────────┘
              └──────────────┘
   IV          ╱
              ▼
  ┌────────┐ ┌───────────────┐   ┌────────┐
  │ Org.LM │▶│ HN-Extraktion │   │ Org.LM │
  └────────┘ └───────────────┘   └────────┘
                    │
                    ▼
  ┌────────┐ ┌───────────────┐   ┌────────┐
  │ Dampf  │▶│ LM-Stripping  │   │ Org.LM │
  └────────┘ └───────────────┘   └────────┘
   V              │
                  ▼
         ┌──────────────────┐
         │ OTS-NH₄-Lsg.     │
         └──────────────────┘         ┌─────────┐
                    ╲                  │ NH₃-Lsg │
                     ▼                 └─────────┘
        ┌──────────────────────────┐  ┌──────────┐
   VI   │       AMINIERUNG         │◀─│ Bisulfit │
        └──────────────────────────┘  │ Lösung   │
                    │                  └──────────┘
  ┌─────────┐       ▼
  │ NaOH-Lg │  ┌──────────────────┐   ┌──────┐
  └─────────┘  │  NH₃-Stripping   │──▶│ NH₃  │
   VII         └──────────────────┘   └──────┘
                    │
                    ▼
        ┌──────────────────┐          ┌──────────┐
  VIII  │  AN-Extraktion   │─────────▶│ AN-Extr. │
        └──────────────────┘          └──────────┘
  ┌────────┐    │
  │ Org.LM │    ▼
  └────────┘ ┌──────────────────────────┐  ┌────────┐
   IX        │      TS-FAELLUNG         │─▶│ H₂SO₄  │
             └──────────────────────────┘  └────────┘
                    │
                    ▼
        ┌──────────────────┐          ┌──────┐
   X    │  SO₂-Desorpt     │─────────▶│ SO₂  │
        └──────────────────┘          └──────┘
                    │
                    ▼
        ┌──────────────────┐
   XI   │  TS-Isolierung   │
        └──────────────────┘
```